# EUROPEAN PATENT APPLICATION

(11) **EP 2 343 074 A1**
(43) Date of publication of application: **13.07.2011**
(21) Application number: 09180688.5
(22) Date of filing: 23.12.2009
(51) Int. Cl.: A61K 31/52, A61K 31/7076, A61P 11/06

(54) **Use of purine analogues for treating airway diseases**

(71) Applicant: Merck Serono S.A., 1267 Coinsins (CH)
(72) Inventor: The designation of the inventor has not yet been filed

(57) **Abstract**

The present invention relates to the treatment and prophylaxis of asthma and other airway dysfunctions, diseases and/or symptoms thereof, more particularly the present invention relates to the use of cytostatic and/or cytotoxic purine analogues for such treatment and prophylaxis.

## Description

### Field of the Invention

The present invention relates to the treatment and prophylaxis of asthma and other airway dysfunctions, diseases and/or symptoms thereof, more particularly the present invention relates to the use of cytostatic and/or cytotoxic purine analogues for such treatment and prophylaxis.

### Background of the Invention

Asthma and other airway dysfunctions or airway diseases and/or its symptoms according to the invention are common and include allergic and/or atopic airway diseases.

Asthma specifically comprises allergic asthma and/or acute severe asthma (ASA, status asthmaticus), severe glucocorticoid-dependent asthma, airway hyperresponsiveness (AHR) in asthma, asthma attacks including acute airway inflammation in asthma and bronchioconstriction associated therewith.

Other airway dysfunctions, diseases and/or symptoms according to the invention include rhinitis, such as allergic rhinitis, rhinosinusitis, sinusitis, chronic obstructive pulmonary disease (COPD), bronchitis, such as chronic bronchitis, emphysema and bronchiectasis or a combination of the conditions as well as airway hyperresponsiveness (AHR) occurring alone or as a part of the diseases, dysfunctions and/or symptoms. These dysfunctions, diseases and/or symptoms are termed in the following each an airway condition or together airway conditions.

Asthma and the other airway conditions are closely related, wherein the allergic airway conditions are characterized by an inappropriate reaction of the respiratory system to stimuli. (Eur Respir J 2006; 28: 264-267, Pediatric Respiratory Reviews (2001) 2, 358-364, Pneumologe 59 (2005), 192- 200, Clinical & Experimental Allergy Reviews 3 (1), 43-45).

In asthma, the airways become blocked or narrowed causing difficulty breathing. Allergic asthma is characterized by symptoms of coughing, wheezing, shortness of breath or rapid breathing, and chest tightness that are triggered by an allergic reaction to inhaled allergens such as dust mite allergen, pet dander, pollen and mold. Symptoms associated with asthma are different for chronic asthma and the acute state of an acute asthma exacerbation or asthma attack and may include breathlessness, wheezing on exhale, dry cough and a feeling of tightness in the chest. These symptoms in patients often can be life threatening. The strong bronchoconstriction and the swelling of the airways can reduce the oxygen and CO₂ exchange in the lungs to an extent where patients die from suffocation. The diagnosis of asthma is frequently based on the measurements of the peak expiratory flow (PEF) rate, which is defined as the maximum airflow during a forced expiration beginning with the lungs fully inflated. Another parameter relevant in asthma is e.g. the forced expiratory volume (FEV), such as the forced expiratory volume in the first second (FEV₁).

Acute severe asthma, ASA or "status asthmaticus", is a devastating clinical condition ultimately resulting in life-threatening hypoxaemia (Eur Respir J 1997; 10: 1359-1371, AMERICAN JOURNAL OF RESPIRATORY AND CRITICAL CARE MEDICINE VOL 168, 2003, 740-759). The abnormalities in pulmonary mechanics can be substantial. The pivotal intrapulmonary mechanism of this condition is profound ventilation/perfusion mismatch, characterized by a predominant bimodal blood flow pattern reflecting a marked deterioration (increase) of the dispersion of pulmonary blood flow. Most patients suffering from acute asthma request therapy with a constellation of complaints consisting of dyspnea, cough, and wheezing.The first two can also occur as isolated entities . The physical signs that are encountered are tachypnea, tachycardia, wheeze, hyperinflation, accessory muscle use, pulsus paradoxus, diaphoresis, cyanosis, and obtundation. The sine qua non of an episode of acute asthma is a nonuniform, reversible increase in airway resistance that results in diminished flow rates, premature airway closure, hyperinflation of the lungs and thorax, increased work of breathing, changes in elastic recoil, and frequency-dependent behavior. In addition, there is abnormal distribution of ventilation and perfusionand altered arterial blood gases. Symptomatic patients with marked obstruction and significant hyperinflation can demonstrate electrocardiographic evidence of pulmonary hypertension, right ventricular strain, hypotension, and poor peripheral perfusion.

Airway hyperresponsiveness (AHR) is a characteristic feature of asthma and consists of an increased sensitivity of the airways to constrictor agonists, as indicated by a smaller concentration of a constrictor agonist needed to initiate the bronchoconstrictor response, a steeper slope of the dose-response, and a greater maximal response to the agonist. (CHEST 2003; 123:411 S-416S). Airway hyperresponsiveness is present in almost all patients with asthma, at least when they are having current symptoms (Thorax 1998;53: 419-424).

Airway responsiveness describes the ability of the airways to narrow after exposure to constrictor agonists. Thus, airway hyperresponsiveness is an increased ability to develop this response. AHR is generally the result of the following components: airway reactivity [slope of increase in total respiratory system resistance for a given increase in constrictor agonists dose], airway sensitivity (lowest dose of constrictor agonists to produce bronchoconstriction), and the maximum inducible bronchoconstriction.

Airway responsiveness is measured using inhalation challenges with airway constrictor agonists, such as histamine or methacholine. Using the method described by Cockcroft et al (Clin Allergy 1977; 7:235-243), asthmatic subjects generally have a provocative concentration of a substance (histamine or methacholine) causing a 20% fall in FEV₁ (PC₂₀) of 8 mg/mL. Most non-asthmatic patients will have a PC₂₀ of 16 mg/mL. The difference in airway responsiveness between normal individuals and asthmatic patients is substantial being 4-8 doubling concentrations of inhaled methacholine less than in those normal subjects in whom it can be measured. In fact, no identifiable airway responsiveness can be measured in many healthy individuals as FEV₁ does not fall by 20%. However, even subjects with normal histamine or methacholine airway responsiveness can develop symptoms of asthma if exposed to specific stimuli to which they are sensitized, such as an inhaled allergen or toluene diisocyanate.

It is well established that patients with more severe asthma have more responsive airways than patients with mild disease, and that patients experiencing exacerbations of asthma—for example, during allergen exposure—develop more pronounced AHR during this period.

Generally, the treatment of asthmatic patients focuses on measurements of asthma symptoms, sleep disturbance, use of rescue medication, limitations of daily activity, lung function as well as patient and physician assessments. The goal of asthma treatment is well-controlled asthma which is generally regarded as including: symptoms occurring twice per week or less, use of a rescue bronchodilator twice a week or less, no nighttime or early morning awakening, no limitations on exercise work or school, normal peak expiratory flow or forced expiratory volume. Complete or total control of asthma symptoms includes: no asthma symptoms and no rescue bronchodilator use as well as no nighttime or early morning awakening and no limitations on exercise, work or school. Well- controlled asthma is generally a realistic target for most but not all patients. Some patients may wish to achieve complete control. However, some may only be able to achieve well-controlled or completely controlled asthma with medications or doses of medications that cause significant adverse effects. There are also certain patients that can only achieve partial control, for example those with steroid- resistant asthma. In treating allergic asthma, the prevention and interference with life threatening bronchioconstriction episodes is a prime goal (J Allergy Clin Immunol. 91 (1993), 97-101).

Several options exist for the treatment of the airway conditions and especially asthma. In general, treatments may be life style modification to reduce the exposure to stimuli, anti-inflammatory medication to relieve the inflammation related symptoms, inflammatory mediator release inhibitors to reduce the effect of single mediators such as histamine or individual interleukins, symptomatic relievers such as anticongestants or bronchodilating agents and finally immune globulins to reduce the immune globulin E driven reaction. With the exception of immune globulin E treatment, the options for achieving control of asthma symptoms require the repeated (daily) prophylactic or metaphylactic administration of drugs.

An acute, or sudden, asthma attack is usually caused by an exposure to allergens or an upper-respiratory-tract infection. The severity of the attack depends on how well the underlying asthma is being controlled. An acute attack is potentially life-threatening because it may continue despite the use of quick-relief medications, such as inhaled bronchodilators.The complicated combination of chronic medications to reduce airway hyperresponsiveness and the frequency and severity of attacks with short acting inhaled drugs to reduce symptoms during an attack makes clear that current treatments are not optimal. Undesirable drug related side effects arise from the use of steroids and beta agonists. The treatment to reduce immune globulin E antibodies is a useful improvement but the intervention must be repeated every few months. As well with the use of antibodies in general, the likelihood is high that the body will identify the administered antibodies as foreign and develops neutralizing antibodies in response. Furthermore, the current treatments focus on the treatment of symptoms but not of the disease or the cause of the disease. Currently there are no treatments available that prevent the allergic reaction from occurring in response to allergen exposure. For other airway dysfunctions, diseases and symptoms included in the airway conditions are also treatment options available, which however usually include repetitive administration of drug in relatively short intervals.

There is thus a medical need to have improved treatments for the airway conditions according to the invention. A therapy that reduces the requirement for daily treatments would be a substantial improvement. Since in many cases the airway conditions exacerbate in form of life-threatening attacks, a prophylaxis to prevent such exacerbation would be highly desirable.

### Summary of the Invention

In one aspect the present invention provides a treatment or a method of treating or the prophylaxis of asthma and other airway dysfunctions or airway diseases and/or symptoms thereof, including allergic asthma, chronic asthma, acute severe asthma (ASA, status asthmaticus), severe glucocorticoid-dependent asthma, airway hyperresponsiveness (AHR) in asthma, asthma attacks including acute airway inflammation in asthma and bronchioconstriction associated therewith, rhinitis, such as allergic rhinitis, rhinosinusitis, sinusitis, chronic obstructive pulmonary disease (COPD), bronchitis, such as chronic bronchitis, emphysema and bronchiectasis or a combination of the conditions as well as airway hyperresponsiveness (AHR) occurring alone or as a part of the diseases, dysfunctions and/or symptoms, by using a cytostatic and/or cytotoxic purine analogue. Especially, the invention relates to a treatment or the prophylaxis of acute severe asthma, severe glucocorticoid-dependent asthma, airway hyperresponsiveness, particularly in ASA, asthma attacks including acute airway inflammation in asthma and bronchioconstriction associated therewith.

Further, the present invention provides the use of a cytostatic and/or cytotoxic purine analogue for the preparation of a medicament for such treatment or prophylaxis.

In an alternative aspect the present invention provides the use of a therapeutically effective amount of a cytostatic and/or cytotoxic purine analogue for the prevention or treatment of asthma and other airway conditions and especially acute severe asthma, severe glucocorticoid-dependent asthma, airway hyperresponsiveness, particularly in ASA, asthma attacks including acute airway inflammation in asthma and bronchioconstriction associated therewith.

### Detailed Description of the Invention

The purine analogues used in the current invention are cytostatic and/or cytotoxic, especially lymphocyte depleting purine and more specifically adenine analogues and are preferably halogenated and/or those of formula I 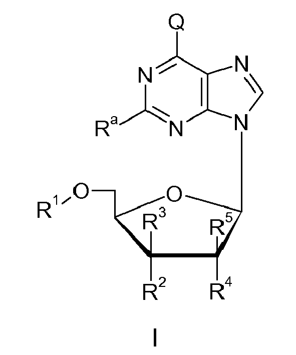 wherein R¹, R², R³, R⁴, R⁵, R^{a} and Q are as defined above
R¹ preferably denotes H or one of the groups -P(O)₂OH, -P(O)₂-P(O)₂OH, or -P(O)₂-P(O)₂-P(O)₂OH or alkali salts thereof, such as the sodium salt thereof. R², R³, R⁴, R⁵ preferably denotes H, OH or Hal. Most preferably the groups are H or OH.
R^{a} preferably denotes H, NH₂ or Hal, most preferrably H or Hal.
Q is OR⁶ or NHR⁶_{.}
R⁶ denotes H, alkyl having 1 to 6 carbon atoms, wherein one H atom may be substituted by an aryl group. Preferably, R⁶ is H, methyl, ethyl, n-propyl, isopropyl or benzyl.
Hal preferably denotes Cl or F

The configuration at the carbon atom of the sugar moiety linked to the nitrogen atom of the purine base of the compounds of formula I and/or the cytostatic and/or cytotoxic purine analoge is preferably beta (beta-D configuration).

Some of the purine analogues are well known e.g. for the treatment of cancers, multiple sclerosis and other diseases (Molecules 2009, 14, 1183-1226). The most preferred purine analogues used according to the invention are the following: cladribine (2-chloro-9-(2'-deoxy-beta-D-ribofuranosyl)-adenine or 2-CdA), fludarabine (2-fluoro-9-(beta-D-arabinofuranosyl)-adenine or FA), pentostatin (2'-deoxycoformycin or DCF), nelarabine (6-methoxy-9-(beta-D-arabinofuranosyl)guanine or pro ara-G), forodesine (7-(3,4-dihydroxy-5-hydroxymethylopyrrolidin-2-yl)-3,5-dihydropyrrolo[3,2-d]pyrimidin-4-one, BCX-1777 or immucillin H), clofarabine (2-chloro-9-(2'-deoxy-2'-fluoro-beta-D-arabinofuranosyl)-adenine or CAFdA), including the respective mono-di- or triphosphates thereof.

The cytostatic and/or cytotoxic purine analogues can be obtained according or in analogy to well known procedures, such as those described in WO2006138396 and WO2004028462.

Cladribine is particularly preferred for use in the present invention.

Cladribine can be formulated in any pharmaceutical preparation suitable for oral administration. Representative oral formulations of 2-CdA are described in (WO 96/19230; WO 96/19229; US 6,194,395; US 5,506,214; WO 2004/087100; WO 2004/087101), the contents of which are incorporated herein by reference.

Processes for preparing 2-CdA are well known in the art. For example, the preparation of 2-CdA is described in (EP 173,059; WO 04028462; WO2006138396; US 5,208,327; WO 0064918) and Robins et al., J. Am. Chem. Soc., 1984, 106: 6379. Alternatively, pharmaceutical preparations of 2-CdA may be purchased from Bedford Laboratories, Bedford, Ohio.

Cladribine or its formulation can be administered orally or parenterally, if its specific bioavailabilty is taken into account. "Administered parenterally" is defined herein as administered in a manner other than through the digestive tract, such as by intravenous subcutaneous or intramuscular injection. Throughout this specification, the parenteral bioavailability is assumend 100%. Therefore, effective doses mentioned herein are also doses suitable for parenteral administration intravenous or intramuscular injection.

Oral administration of cladribine is preferred and may be in capsule, tablet, oral suspension, or syrup form. The tablet or capsules may contain from about 3 to 500 mg of cladribine. Preferably they may contain about 3 to about 10 mg of cladribine, more preferably about 3, about 5 or about 10 mg of cladribine. The capsules may be gelatin capsules and may contain, in addition to cladribine in the quantity indicated above, a small quantity, for example less than 5% by weight, magnesium stearate or other excipient. Tablets may contain the foregoing amount of the compound and a binder, which may be a gelatin solution, a starch paste in water, polyvinyl polyvinyl alcohol in water, etc. with a typical sugar coating.

Liquid formulations for injection may be obtained e.g. according to US5,641,757 or US 5,681,822.

Liquid and dry powder formulations may also be inhaled. Inhalation requires significant less drug to achieve a pharmaceutical effect, due to the high concentration achieved locally in the airways.

In one embodiment, cladribine is used in the treatment or prophylaxis of asthma according to the invention, wherein the oral administration of cladribine to an individual in need thereof comprises the following steps:
(i) An induction treatment wherein the total dose of cladribine reached at the end of the induction period is period is about 0.75 mg/kg to about 3.5 mg/kg, e.g. about 1.7 mg/kg to about 3.5 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

In another embodiment, cladribine is used in the treatment or prophylaxis of asthma according to the invention, wherein the oral administration of cladribine to an individual in need thereof comprises the following steps:
(i) An induction treatment wherein the total dose of cladribine reached at the end of the induction period is period is about 0.75 mg/kg to about 1.25 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

In another embodiment, cladribine is used in the treatment or prophylaxis of asthma according to the invention, wherein the administration via injection of cladribine to an individual in need thereof comprises the following steps:
(i) An induction treatment wherein the total dose of cladribine reached at the end of the induction period is period is about 0.3 mg/kg to about 1.4 mg/kg, e.g. about 0.7 mg/kg to about 1.4 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

In another embodiment, cladribine is used in the treatment or prophylaxis of asthma according to the invention, wherein the administration via injection of cladribine to an individual in need thereof comprises the following steps:
(i) An induction treatment wherein the total dose of cladribine reached at the end of the induction period is period is about 0.3 mg/kg to about 0.5 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

The terms "about 0.3 mg/kg", "about 0.5 mg/kg", "about 0.7 mg/kg", "about 0.75 mg/kg", "about 1.25 mg/kg", about 1.7 mg/kg", "about 1.75 mg/kg", "about 2.1 mg/kg", about 3.5 mg/kg" and other doses given are preferably defined as the values having a maximum negative or positive deviation of about 5 to about 30 percent, more preferably of about 5 to about 30 percent and even more preferably of about 5 to about 10 percent.

The "total dose" or refers to the total dose of cladribine administered during the treatment, i.e. the dose reached at the end of the treatment period, ie. the induction period or maintenance period, that is calculated by adding the daily doses. For example, the total dose of cladribine corresponding to a treatment of 0.7 mg/kg cladribine per day during 5 days is 3.5 mg/kg or the total dose of cladribine corresponding to a treatment of 0.35 mg/kg cladribine per day during 5 days is 1.75 mg/kg.

Typically, the bioavailability of cladribine or of a cladribine formulation used in the context of this invention is from about 30% to about 90%, preferably from about 40% to about 60%, such as about 50%.

An induction period lasts up to about 4 months or up to about 3 month or up to about 2 months. For example, an induction period lasts for about 2 to about 4 months. An induction period consists in the oral administration of cladribine or a pharmaceutical preparation thereof during about 1 to about 7 days each month.

A maintenance period lasts for up to about 4 months, or up to about 3 months, or up to about 2 months, preferably up to about 2 months. For example, a maintenance period lasts for about 2 to about 4 months, preferably for about 2 months. A maintenance period consists in the oral administration of cladribine or a pharmaceutical preparation thereof during about 1 to about 7 days each month.

A "cladribine-free period" is a period wherein no cladribine is administered to the patient. During a cladribine-free period, the patient can be free of any administration or be dosed with a placebo-pill or another drug. A cladribine-free period lasts at least 8 months. Generally it lasts up to about 10 months or up to 9months . For example, a cladribine-free period lasts from about 8 to about 10 months, typically at least of about 8 months.

Cladribine or a pharmaceutical preparation thereof is administered during about 1 to about 7 days each month.

The present invention provides a method of of a cytostatic purine analogue for inhibiting an airway condition comprising administering to a subject suffering from asthma or other airway conditions, a therapeutically effective amount of a cytostatic and/or cytotoxic purine analogue.

Adenosine and adenosine analogs are known to be active on adenosine receptors such as A1, A2A, A2B and A3, in particular A2B receptors play an important role in mast cell activation and have potential relevance for the treatment of asthma (Trends in Pharmacological Sciences, Volume 19, Issue 4, 1 April 1998, Pages 148-153). However, suggested compounds are not cytostatic and/or cytotoxic.

It is also known that adenosine can cause bronchoconstriction (Thorax. 1997 March; 52(3): 239-243).

In an alternative aspect, the present invention provides the use of a therapeutically effective amount of a purine analogue for the prevention or treatment of asthma and other airway conditions and especially acute severe asthma, severe glucocorticoid-dependent asthma, airway hyperresponsiveness, particularly in ASA, asthma attacks including acute airway inflammation in asthma and bronchioconstriction associated therewith by administering a therapeutically effective amount of a cytostatic and/or cytotoxic purine analogue.

It will be understood from the description, and the examples provided, that the cytostatic purine analogue may be administered in patients who are free of a specific airway condition to prevent the occurrence and re-occurrence of this airway condition, in patients with mild symptoms, or even in patients with moderate to severe symptoms.

Patients may be drug free or drug naïve, or may be receiving any other medication in addition to the cytostatic and/or cytotoxic purine analog according to the invention, which are known to treat the airway conditions.

The present invention is particularly suitable, if the asthma patient is free of viral infection.

The cytostatic and/or cytotoxic purine analogue is preferably administered to the subject orally. Intravenous administration or inhalation of the purine analogue is also possible. The cytostatic and/or cytotoxic purine analogue may be co-administered or combined with all currently known drugs used in asthma and other airway conditions, such as anti-allergic medications, given orally, parenterally, topically, via transdermal therapeutic systems, rectally or by inhalation. These medications, as mentioned above, include steroids (inhaled, intranasal, as eye drops, orally or via parenteral, rectal or other routs), antihistamines, beta-agonists, anti-muscarine drugs, immune modulators such as PDE4 inhibitors, leukotrien antagonists, anti-IgE antibodies, and other (innovative) medications.

Specifically, the cytostatic and/or cytotoxic purine analogue may be co-administered or combined with ciclesonide, beclomethasone, budesonide, flunisolide, fluticasone, mometasone, and triamcinolone, montelukast, zafirlukast, pranlukast, and zileuton, cromoglicate (cromolyn), nedocromil, ipratropium, oxitropium, and tiotropium, methylxanthines (theophylline and aminophylline) and/or omalizumab. Furthermore, the cytostatic and/or cytotoxic purine analogue used in the present invention may be co-administered or combined with immunosuppressive drugs, such as S1 P1 agonists, specifically FTY720 (fingolimod), methotrexate or cyclosporine, which may be useful in the treatment of asthma (The Journal of Clinica Investigation, Vol116, No. 11, 2006, 2935-2944, Eur Respir J 2000, 15: 478-485, Int Arch Allergy Immunol 1992; 99: 284-288).

The co-administration or combination of the cytostatic and/or cytotoxic purine analogue with steroids, especially glucocorticoids, such as ciclesonide, beclomethasone, budesonide, flunisolide, fluticasone, mometasone, and triamcinolone, is particularly preferred, and even more preferred, if the glucocorticoids are inhaled.

In an alternative aspect the present invention therefore provides the use of a therapeutically effective amount of a purine analogue for the prevention or treatment of severe glucocorticoid-dependent asthma by administering a therapeutically effective amount of a cytostatic and/or cytotoxic purine analogue.

In an alternative aspect the present invention therefore provides the use of a therapeutically effective amount of a purine analogue for reducing the necessary amount of glucocoticoids in severe glucocorticoid-dependent asthma by administering a therapeutically effective amount of a cytostatic and/or cytotoxic purine analogue.

The cytostatic and/or cytotoxic purine analogue may also be administered as monotherapy.

The cytostatic and/or cytotoxic purine analogue can be administered according to the invention in various ways to the patient. Suitable treatment regimens are e.g. disclosed in US5506214 or Expert Rev Anticancer Ther 8(4) 535-545 (2008). A preferred embodiment uses cladribine in a treatment regimen according to WO2006067141. Although the treatment regimens described in the prior art are usually intended for the treatment of multiple sclerosis and cancer, they can also be used for the purposes of the present invention.

Further, the described use of cytostatic purine analogues also comprise the use of cytostatic purine analogues in the manufacture or preparation of a medicament for the treatment described in the present application.

Methods of treatment and prophylaxis including the foregoing uses are also object of the invention.

It will be understood that the present invention is directed towards the treatment of a subject that includes mammals, for example humans, horses, cats and dogs.

The following examples serve to illustrate preferred embodiments of the present invention. Those skilled in the art will recognize that various modifications may be made to the foregoing description and the following examples and that the following examples are not meant to be limiting with respect to the scope of the invention.

### Example 1:

Female BALB/c mice, aged 10-12 wk, are housed in environmentally controlled specific pathogen-free conditions for 1 wk before study and for the duration of the experiments, which are performed according to J Appl Physiol, VOL 97, 2004, 2258-2265. Accordingly, mice are sensitized with intraperitoneal ovalbumin conjugated to aluminum potassium sulfate, as described in Am J Respir Cell Mol Biol 21: 473-479, 1999. Sensitized mice are subjected to either brief or chronic periods of allergen exposure, as described in Am J Respir Cell Mol Biol 27: 526-535, 2002. Control mice are subjected to the same sensitization protocol but are receiving saline challenges.

Allergen-challenged and control mice are studied at 24 h after the final exposure (at day 21) in the brief allergen-challenge protocol and at week 4 after the final exposure in the chronic allergen-challenge protocol. Intervention is performed in parallel by using cladribine in a phosphate-buffered solution or a phosphate-buffered solution without cladribine as control, which is administered intraperitoneally on days 1, 5 and 9 of the study, every 3 hours a day at a dose of 15 mg/kg body weight per injection according to Proc. Natl. Acad. Sci. USA, Vol. 77, No 11, pp. 6865-6869.

Separate groups of 10 mice are studied in each treatment arm of the protocol.

At day 21 or week 4, respectively, the underlying contributors to airway responsiveness, namely airway reactivity [slope of increase in total respiratory system resistance (Rrs) for a given increase in MCh dose], airway sensitivity (lowest dose of MCh to produce bronchoconstriction), and the maximum inducible bronchoconstriction (maximum Rrs) are measured following brief or chronic allergen challenge. These components of airway responsiveness are measured based on the response of Rrs to saline and increasing (10, 33, 100, and 330 µg/kg) intravenous doses of MCh. Rrs is measured by using the flow interrupter technique, modified for use in mice, and described in Am J Respir Cell Mol Biol 21: 473-479, 1999, Am J Respir Cell Mol Biol 27: 526-535, 2002 and J Appl Physiol 89: 413-421, 2000. Total respiratory system resistance (Rrs) is measured in response to increasing doses of intravenous methacholine (MCh). Using the resulting Rrs-MCh dose-response curve, indexes of airway reactivity (Slope Rrs), airway sensitivity, or the lowest dose to produce bronchoconstriction (Break Rrs) and maximal degree of bronchoconstriction (Max Rrs) are measured.

Furthermore, total and differenctial cell counts in bronchoalveolar lavage fluid are performed according to Am J Respir Cell Mol Biol 21: 473-479, 1999.

Comparisons with respect to airway reactivity (slope of the Rrs-log transformed MCh dose-response curve), maximal bronchoconstriction (maximal MCh-induced Rrs), cell counts, between saline control mice and mice receiving either brief or chronic allergen exposure, being treated with either cladribine or phosphate buffer solution without cladribine, are made.

The experiment shows that treatment with cladribine during allergen exposure reduces or abrogates asthma related symptoms such as increased bronchoconstriction and allergen-induced development of AHR that is present in mice receiving allergen-challenge and phosphate buffer (without cladribine). Thus, cladribine treatment is beneficial to reduce signs of asthma, increased airway reactivity and maximal bronchoconstriction, as compared to the control. In addition, little or no host toxicity is resulting from the use of cladribine.

### Example 2

A patient having asthma with recurring asthma attacks is treated with oral cladribine tablets and following the dosage regimen disclosed in WO2006067141. As a result, the symptoms of asthma and airway hyperresponsiveness are reduced in this patient.

## Claims

1. A cytostatic and/or cytotoxic purine analogue for use in the treatment or prophylaxis of asthma and other airway dysfunctions or airway diseases and/or symptoms thereof.

2. A cytostatic and/or cytotoxic purine analogue for use in the treatment or prophylaxis of acute severe asthma, severe glucocorticoid-dependent asthma, airway hyperresponsiveness, particularly in ASA, asthma attacks including acute airway inflammation in asthma and bronchioconstriction associated therewith.

3. A cytostatic and/or cytotoxic purine analogue for use in the treatment or prophylaxis of asthma according to claims 1 or 2, wherein the purine analogue is co-administered or combined with steroids, antihistamines, beta-agonists, anti-muscarine drugs, immune modulators, leukotrien antagonists and/or anti-IgE antibodies and/or immunosuppressants.

4. A cytostatic and/or cytotoxic purine analogue for use in the treatment or prophylaxis of asthma according to any of the foregoing claims, wherein the purine analogue is administered orally or by inhalation.

5. A cytostatic and/or cytotoxic purine analogue for use in the treatment or prophylaxis of asthma according to any of the foregoing claims, wherein the purine analogue is cladribine (2-chloro-9-(2'-deoxy-beta-D-ribofuranosyl)-adenine or 2-CdA), fludarabine (2-fluoro-9-(beta-D-arabinofuranosyl)-adenine or FA), pentostatin (2'-deoxycoformycin or DCF), nelarabine (6-methoxy-9-(beta-D-arabinofuranosyl)guanine or pro ara-G), forodesine (7-(3,4-dihydroxy-5-hydroxymethylopyrrolidin-2-yl)-3,5-dihydropyrrolo[3,2-d]pyrimidin-4-one, BCX-1777 or immucillin H), clofarabine (2-chloro-9-(2'-deoxy-2'-fluoro-beta-D-arabinofuranosyl)-adenine or CAFdA), as well as the respective mono-di- or triphosphates thereof.

6. A cytostatic and/or cytotoxic purine analogue for use in the treatment or prophylaxis of asthma according to any of the foregoing claims, wherein the purine analogue is cladribine (2-chloro-9-(2'-deoxy-beta-D-ribofuranosyl)-adenine or 2-CdA).

7. Cladribine for use in the treatment or prophylaxis of asthma, wherein the oral administration of cladribine to an individual in need thereof comprises the following steps:
(i) An induction treatment wherein the total dose of cladribine reached at the end of the induction period is period is about 0.75 mg/kg to about 3.5 mg/kg, e.g. about 1.7 mg/kg to about 3.5 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

8. Cladribine for use in the treatment or prophylaxis of asthma, wherein the oral administration of cladribine to an individual in need thereof comprises the following steps:
(i) An induction treatment wherein the total dose of cladribine reached at the end of the induction period is period is about 0.75 mg/kg to about 1.25 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

9. Cladribine for use in the treatment or prophylaxis of asthma, wherein the administration via injection of cladribine to an individual in need thereof comprises the following steps:
(i) An induction treatment wherein the total dose of cladribine reached at the end of the induction period is period is about 0.3 mg/kg to about 1.4 mg/kg, e.g. about 0.7 mg/kg to about 1.4 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.

10. Cladribine for use in the treatment or prophylaxis of asthma, wherein the administration via injection of cladribine to an individual in need thereof comprises the following steps:
(i) An induction treatment wherein the total dose of cladribine reached at the end of the induction period is period is about 0.3 mg/kg to about 0.5 mg/kg;
(ii) A cladribine-free period wherein no cladribine is administered;
(iii) A maintenance treatment wherein the total dose of cladribine reached at the end of the maintenance period is equal to or lower than the total dose of cladribine reached at the end of the induction period (i);
(iv) A cladribine-free period wherein no cladribine is administered.
